# EUROPEAN PATENT SPECIFICATION

(11) **EP 3 743 141 B1**
(45) Date of publication and mention of the grant of the patent: **08.02.2023**
(21) Application number: 18830315.0
(22) Date of filing: 18.12.2018
(51) Int. Cl.: A61M 16/04, A61M 16/08

(54) **DEVICE AND KIT FOR RETROGRADE INTUBATION**
VORRICHTUNG UND KIT ZUR RETROGRADEN INTUBATION
DISPOSITIF ET KIT D'INTUBATION RÉTROGRADE

(30) Priority: 23.01.2018 IT 201800001653
(43) Date of publication of application: 02.12.2020
(73) Proprietor: GIMAC INTERNATIONAL S.r.l., 21040 Castronno (VA) (IT)
(72) Inventor: MUNARO, Marco, 21040 Castronno (VA) (IT)
(74) Representative: Gregorj S.r.l.
(86) International application number: PCT/IB2018/060256
(87) International publication number: WO 2019/145774

(56) References cited:
- CN-Y- 201 361 337
- US-A- 5 507 279
- US-A1- 2009 107 509
- US-A1- 2016 038 008

## Description

### Technical field of the invention

The present invention refers to a device and kit for the retrograde intubation.

### Prior art

Several techniques for the intubation for the purpose of assisting and controlling the airways of a patient are known.

A first technique, known as endotracheal intubation, consists of introducing into the patient mouth a laryngoscope, for enabling a paramedic to see the patient glottis and, afterwards, of introducing a ventilation tube. However, such technique requires to subject the patient head to movements, which can be dangerous, particularly in the presence of serious traumas.

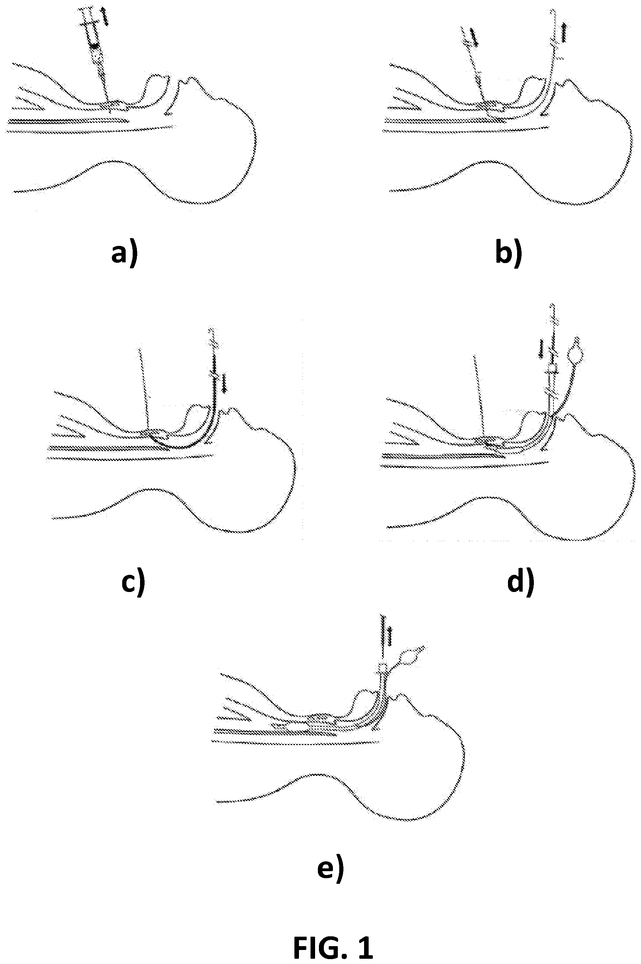

There are several alternative techniques especially in case of a problematic intubation, one among them is the so-called retrograde intubation. Figures from 1a to 1e schematically illustrate the steps of such technique. It comprises:
- making a hole, for example by a needle, through the patient cricothyroid or cricotracheal membrane (Figure 1a);
- introducing a guide wire through the made hole and extracting it from the patient mouth (Figure 1b);
- according to a possible variant of the technique, positioning on the guide wire an anterograde guide and sliding the latter on the same (Figure 1c);
- introducing through the mouth of the patient an endotracheal tube on the anterograde guide, if used, or directly on the guide wire (Figure 1d);
- extracting the guide wire and/or anterograde guide for enabling to advance the endotracheal tube and then ventilating the patient (Figure 1e).

However, also this retrograde intubation technique, even though does not require substantial movements of the patient head, exhibits some possible disadvantages. Particularly, when it is introduced the endotracheal tube (Figure 1d), this can encounter obstructions, such as the vocal cords or epiglottis, for example. Moreover, if the endotracheal tube is advanced after removing the guide wire (Figure 1e), the endotracheal tube can uncorrectly advance. Besides that, the retrograde intubation kits comprise, as discussed, several components which both increase the complexity and the overall cost.

Known devices for retrograde intubation are disclosed in documents US 2009/107509 A1, US 5 507 279 A, US 2016/038008 A1 and CN 201 361 337 Y.

### Summary of the invention

Therefore, an object of the present invention consists of making available a device and a kit for the retrograde intubation, which reduces the risk of problems during the intubation itself and which are simple, in order to be sufficiently inexpensive.

This and other objects are obtained by a device for the retrograde intubation according to the present invention, as disclosed by claim 1, and by a kit for the retrograde intubation according to the present invention, as disclosed by claim 10.

### Brief description of the drawings

In order to gain a better comprehension of the invention and appreciating the advantages, some exemplifying non-limiting embodiments thereof will be described in the following with reference to the attached drawings, wherein:
Figures from 1a to 1e illustrate the steps of a retrograde intubation, performed by a kit according to the prior art;
Figures from 2a to 2f illustrate possible steps of a retrograde intubation, performed by a retrograde intubation kit according to the invention;
Figure 3 is a cross-section schematic view of a retrograde intubation device according to a possible embodiment;
Figure 4 is a cross-section schematic view of a retrograde intubation device according to a further embodiment.

### Detailed description of the invention

With reference to the attached Figures from 2 to 4, a retrograde intubation device is generally indicated by the reference number 1.

The device 1 comprises a guide wire 2 having a longitudinal extension so that can be inserted through a hole made in the cricothyroid or cricotracheal membrane of a patient and can exit the mouth or nose of the patient, meanwhile some portions are maintained outside the length between the made hole and the patient mouth. The guide wire 2 exhibits characteristics which enable it to inflect along such insertion for adapting to the actual path. For example, the guide wire 2 can be made of a metal or polymer material.

Moreover, the device 1 comprises a flexible tube 3 fixedly connected to the guide wire 2, so that the longitudinal movements of the flexible tube 3 with respect to the guide wire 2 are prevented. The flexible tube 3 longitudinally extends parallel to the guide wire 2 and is shaped so that:
- it inflects following the inflections of the guide wire 2;
- it radially deforms upon its insertion through holes having a radial extension less than the radial extension of the tube 3 itself, particularly a hole made in the cricothyroid or cricotracheal membrane of the patient.

The flexible tube 3 has a longitudinal extension less than the longitudinal extension of the guide wire 2, so that the guide wire 2 exhibits at least one end portion 4 where the guide wire is devoid of the flexible tube 3. According to a possible embodiment, the flexible tube 3 is longitudinally arranged in an intermediate position of the guide wire 2, so that the guide wire 2, in addition to said first end portion 4, moreover exhibits a second end portion 5, opposite to the first one 4, where the guide wire 2 is devoid of the flexible tube 3.

According to a possible embodiment (not illustrated in the figures), the flexible tube 3 comprises one or more openings/holes in proximity of the distal end, in other words the one destined to be placed inside the trachea, opposite to the first end portion 4 of the guide wire 2. These openings/holes enable air/oxygen to flow through the flexible tube 4 towards the lungs if the terminal port of the flexible tube 3 is obstructed or stuck into the cutaneous-subcutaneous tissue. Advantageously, at least one of these openings/holes has a diameter sufficient to enable a known-type tracheal tube to pass through.

According to a possible embodiment, the flexible tube 3 is made of a material selected in the group consisting of polyvinyl chloride (PVC), polyurethane, thermoplastic elastomers (TPE), polyethylene (PE), polypropylene (PP), polyether block amide (PEBA), polyamide (PA). Moreover, it can be coated on the external surface and/or internal surface by a hydrophilic material. The thickness of the flexible tube 3 depends on the used material and is selected so that the flexible tube 3 has the mechanical characteristics, particularly the hereinbefore described axial and radial deformabilities. Preferably, such thickness is comprised from 50 to 500 microns.

Preferably, the flexible tube 3 is longitudinally constrained to the guide wire 2 at a circumferential wall 6 thereof forming the tube itself.

Coupling the flexible tube 3 to the guide wire 2 is made according to different modes.

According to a possible embodiment, the flexible tube 3 is glued to the guide wire 2 along all the longitudinal length thereof or at a plurality of points. Gluing is alternatively made inside or outside the flexible tube 3.

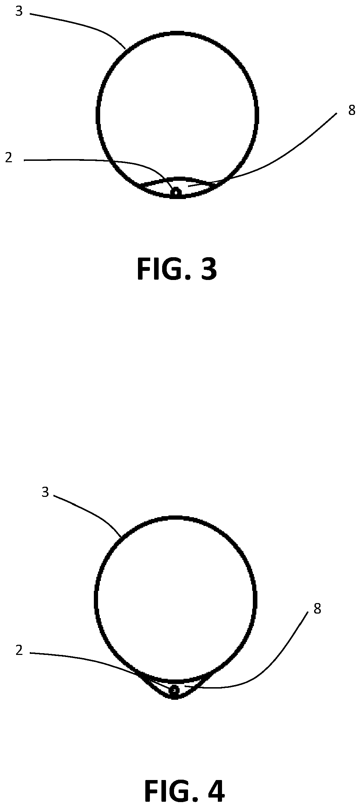

According to a further possible embodiment, the flexible tube 3 comprises a seat 8 inside (Figure 3) or outside (Figure 4) the flexible tube 3 itself, for example in the shape of a lap adapted to receive the guide wire 2. This latter can be constrained in the seat by gluing, or by melting the flexible tube 3 along all the length thereof or at a plurality of points. As an alternative, the guide wire 2 can comprise locking means adapted to constrain the flexible tube 3 to the guide wire 2 itself. For example, such locking means can comprise shaped protrusions adapted to generate friction on the flexible tube 3 or, alternatively, adapted to lock it by holes made in the same. According to a further possible embodiment, said seat 8 can be obtained by a sub-channel longitudinally extending in the flexible tube.

According to a further possible embodiment, the guide wire 2 and flexible tube 3 can be simultaneously co-extruded, and therefore they can be longitudinally coupled and constrained. According to this variant, the guide wire 2 and flexible tube 3 for example can be both made of a polymeric material.

Advantageously, the flexible tube 3 has a size so that the interior thereof can longitudinally slidingly receive a known-type endotracheal tube 7. Further, the flexible tube 3 has preferably a longitudinal size less than the overall longitudinal extension of such endotracheal tube 7. If the longitudinal size of the flexible tube 3 is not less than the one of the endotracheal tube 7 inserted in the interior thereof, it is possible to cut the surplus portion of the flexible tube 3, which could make difficult to insert the endotracheal tube 7 in the interior thereof.

Advantageously, the material and thickness of the flexible tube 3 are designed so the flexible tube 3 does not break or tear when the endotracheal tube 7 is introduced in the interior thereof.

Still more advantageously, at least one of the ends of the flexible tube 3 is shaped so that it enables to connect the flexible tube 3 itself to a known ventilation system 9 directly or by an intermediate connecting device. For example, the end of the flexible tube 3 can have a radial size so that it is connected by an interference fit to a joining portion of the ventilation system 9 or intermediate connecting device or, alternatively, can be corrugated for obtaining a friction fit.

The device 1 according to the invention and the kit comprising the device 1 and endotracheal tube 7 can be made with different sizes so that can be used both with adults and kids.

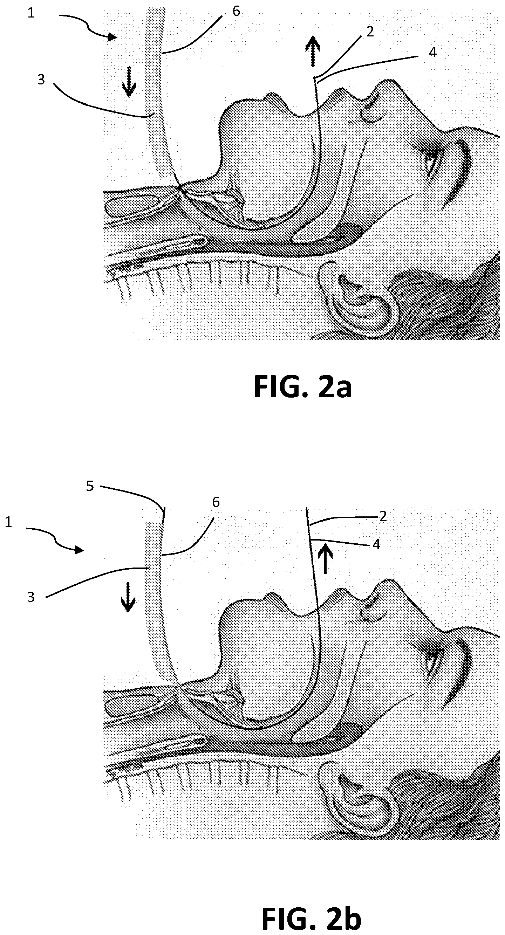

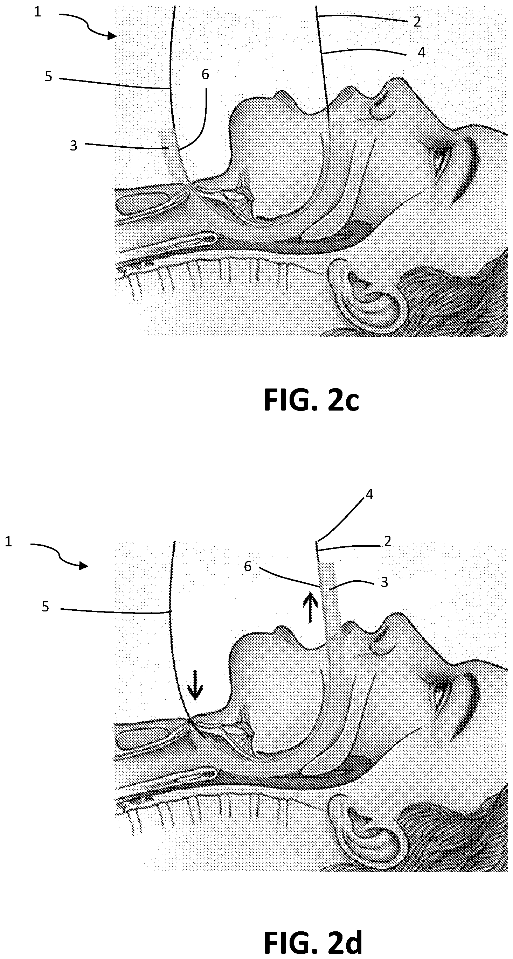

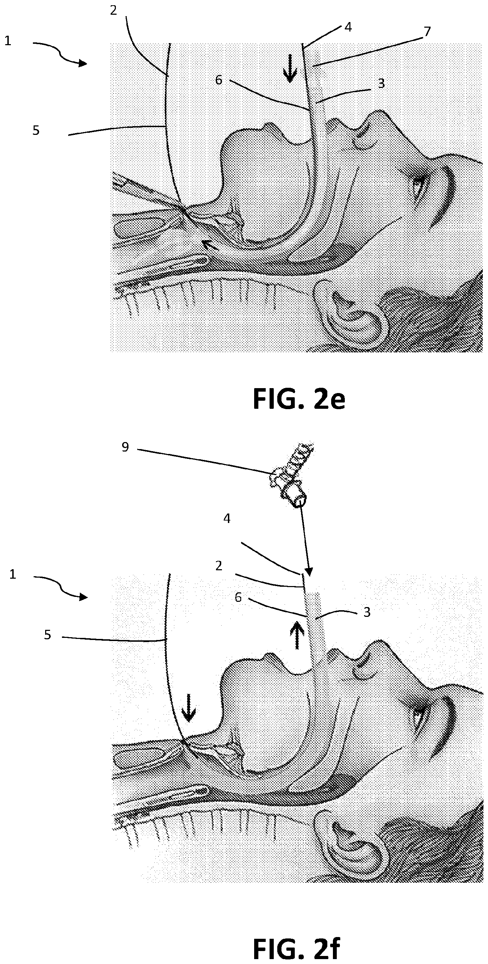

The steps for a retrograde intubation performed by the kit according to the invention will be described in the following.
- Upon making a hole, for example by a needle, through the cricothyroid or cricotracheal membrane of the patient, the first end portion 4 of the guide wire 2 is inserted through the hole itself (Figure 2a);
- then the wire guide 2 is further inserted through the hole, sliding inside the trachea in the caudal-cranial direction until it exits the patient mouth (Figure 2b), or, alternatively, the nose. In turn, the flexible tube 3 encounters the hole which has a smaller radial size, so that it radially deforms;
- then the guide wire 2 is kept sliding (Figure 2c) with the flexible tube 3 integral to it, until the flexible tube 3 exits the patient mouth or nose and no portion thereof remains outside the hole (Figure 2d);
- then the endotracheal tube 7 is introduced by sliding it inside the flexible tube portion 3 projecting from the patient mouth, until it exits from the end of the flexible tube 3 housed inside the trachea (Figure 2e). In this arrangement, the endotracheal tube 7 is made sliding besides the end of the flexible tube 3 to the interior of the trachea which is already in the correct position, for starting the ventilation by a common-type ventilation system.
- As an alternative to what was beforehand illustrated with reference to Figure 2e, the step of introducing the endotracheal tube 7 can be omitted and the distal portion of the flexible tube 3 exiting the mouth or nose can be connected to a common ventilation system 9 (Figure 2f), since the flexible tube 3 is also a channel through which air and oxygen are blown into the patient airways. The connection of the flexible tube 3 to the ventilation system 9 can be directly made or by an intermediate joining device enabling to connect the flexible tube 3 to the ventilation system 9.

According to a further variant not illustrated in the figures, it is observed that the flexible tube 3 can be made so that the proximal and distal ends thereof are closed, for example, are occluded by the same material which the flexible tube 3 is made of. In such arrangement, the flexible tube 3 forms an elongated balloon capable of helping it to pass through the hole made in the cricothyroid or cricotracheal membrane of the patient. The two ends of the flexible tube 3 will be successively opened. For example, they can be cut, or, alternatively, they can be provided with a preferably transversal weakening line, so that some material can be removed. Consequently, the internal channel of the flexible tube 3 is opened anew so that it can receive the tracheal tube or can be connected to the ventilation system, according to what was hereinbefore described.

According to a further embodiment variant not illustrated in the figures, the kit can comprise a per se known cuff provided with a cuff inflating-deflating channel. Such cuff can be applied to the flexible tube 3 for example at the end thereof corresponding to the end 5 of the guide wire 2. The cuff inflating-deflating channel can extend towards the end 4 or end 5 of the guide wire. This enables to make more stable the kit inside the airway and to protect the same from different kinds of materials, for example organic materials, which can possibly enter inside. Advantageously, the cuff can be blown after introducing a known-type tracheal tube inside the flexible tube 3 or generally any other element capable of maintaining its own diameter, for preventing the flexible tube 3 from radially excessively deforming and therefore eliminating the risk of an obstruction thereof.

From what was hereinbefore described, it is understood that the retrograde intubation performed by the kit according to the invention does not require to remove the guide wire 2, which is always present and therefore enables to easily insert the endotracheal tube or, alternatively, to ventilate the patient without the necessity to use the endotracheal tube. Generally the operation requires a limited number of steps and the kit comprises a limited number of components.

## Claims

1. Device (1) for the retrograde intubation, comprising a guide wire (2) and a flexible tube (3) having a longitudinal extension less than the one of the guide wire (2), wherein the flexible tube (3) longitudinally extends parallel to the guide wire (2) and is shaped for inflecting by following the inflections of the guide wire (2) and for radially deforming upon inserting it in a hole made in the cricothyroid or cricotracheal membrane of a patient having a radial extension less than the radial extension of the flexible tube (3), **characterized in that** the flexible tube (3) is fixedly connected to the guide wire (2) and has a size so that an endotracheal tube (7) can be longitudinally inserted and slid in the interior thereof.

2. Device (1) for the retrograde intubation according to claim 1, wherein the flexible tube (3) is arranged on the guide wire (2) so that the guide wire (2) has at least one first end portion (4), where the guide wire (2) is devoid of the flexible tube (3).

3. Device (1) for the retrograde intubation according to claim 1 or 2, wherein the flexible tube (3) is arranged in an intermediate longitudinal position of the guide wire (2), so that the guide wire (2) has a first end portion (4) and a second end portion (5) opposite to the first one (4), wherein the guide wire (2) is devoid of the flexible tube (3).

4. Device (1) for the retrograde intubation according to anyone of the preceding claims, wherein the flexible tube (3) is made of a material selected in the group consisting of: polyvinyl chloride (PVC), polyurethane, thermoplastic elastomers (TPE), polyethylene (PE), polypropylene (PP), polyether block amide (PEBA), polyamide (PA).

5. Device (1) for the retrograde intubation according to anyone of the preceding claims, wherein the flexible tube (3) has a thickness comprised between 50 and 500 microns.

6. Device (1) for the retrograde intubation according to anyone of the preceding claims, wherein the flexible tube (3) is longitudinally constrained to the guide wire (2) at a circumferential wall (6) thereof.

7. Device (1) for the retrograde intubation according to anyone of the preceding claims, wherein at least one of the ends of the flexible tube (3) is shaped for enabling to connect the flexible tube (3) to a ventilation system (9) directly or by an intermediate connecting device.

8. Device (1) for the retrograde intubation according to anyone of the preceding claims, wherein said flexible tube (3) comprises one or more openings/holes positioned in proximity of the distal end, enabling the air to pass through.

9. Device (1) for the retrograde intubation according to anyone of the preceding claims, wherein the flexible tube (3) is made so that the proximal and distal ends thereof are closable and openable by removing some material.

10. Kit for the retrograde intubation comprising:
- a device (1) for the retrograde intubation according to anyone of the preceding claims;
- an endotracheal tube (7) adapted to be longitudinally inserted and slid inside the flexible tube (3) of said device (1), and/or an intermediate connecting device for directly connecting the flexible tube (3) to a ventilation system (9).

11. Kit for the retrograde intubation according to claim 10, wherein the flexible tube (3) of the device (1) has a longitudinal size less than the overall longitudinal extension of the endotracheal tube (7).

12. Kit for the retrograde intubation according to claim 10 or 11, further comprising a ventilation system (9) connectable to the flexible tube (3) and/or to the endotracheal tube (7) and/or to the intermediate connecting device.

13. Kit for the retrograde intubation according to anyone of claims from 10 to 12, further comprising a cuff connectable to the flexible tube (3), and provided with a cuff inflating-deflating channel, so that said cuff inflating-deflating channel can extend towards the first (4) or second end (5) of the guide wire (2).

## Patentansprüche

1. Vorrichtung (1) für die retrograde Intubation, umfassend einen Führungsdraht (2) und einen flexiblen Tubus (3), der eine geringere Längserstreckung als der Führungsdraht aufweist (2), wobei sich der flexible Tubus (3) in Längsrichtung parallel zum Führungsdraht (2) erstreckt und dazu ausgebildet ist sich zu biegen, indem er den Biegungen des Führungsdrahtes (2) folgt, und sich beim Einführen in ein Loch, das in der Krikothyreoidea oder Krikotrachealmembran eines Patienten ausgebildet ist, radial zu verformen, wobei seine radiale Erstreckung geringer ist als die radiale Erstreckung des flexiblen Tubus (3), **dadurch gekennzeichnet, dass** der flexible Tubus (3) fest mit dem Führungsdraht (2) verbunden ist und eine solche Größe aufweist, dass ein Endotrachealtubus (7) in Längsrichtung eingeführt und ins Inneren davon geschoben werden kann.

2. Vorrichtung (1) für die retrograde Intubation nach Anspruch 1, wobei der flexible Tubus (3) so an dem Führungsdraht (2) angeordnet ist, dass der Führungsdraht (2) mindestens einen ersten Endabschnitt (4) aufweist, an dem der Führungsdraht (2) frei von dem flexiblen Tubus (3) ist.

3. Vorrichtung (1) für die retrograde Intubation nach Anspruch 1 oder 2, wobei der flexible Tubus (3) in einer mittleren Längsposition des Führungsdrahtes (2) angeordnet ist, so dass der Führungsdraht (2) einen ersten Endabschnitt (4) und einen dem ersten (4) gegenüberliegenden zweiten Endabschnitt (5) aufweist, an dem der Führungsdraht (2) frei von dem flexiblen Tubus (3) ist.

4. Vorrichtung (1) für die retrograde Intubation nach einem der vorhergehenden Ansprüche, wobei der flexible Tubus (3) aus einem Material besteht, das aus der Gruppe ausgewählt ist, die aus Folgendem besteht: Polyvinylchlorid (PVC), Polyurethan, thermoplastischen Elastomeren (TPE), Polyethylen (PE), Polypropylen (PP), Polyetherblockamid (PEBA) und Polyamid (PA).

5. Vorrichtung (1) für die retrograde Intubation nach einem der vorhergehenden Ansprüche, wobei der flexible Tubus (3) eine Dicke aufweist, die zwischen 50 und 500 Mikrometer umfasst.

6. Vorrichtung (1) für die retrograde Intubation nach einem der vorhergehenden Ansprüche, wobei der flexible Tubus (3) in Längsrichtung an einer Umfangswand (6) des Führungsdrahts (2) befestigt ist.

7. Vorrichtung (1) für die retrograde Intubation nach einem der vorhergehenden Ansprüche, wobei mindestens eines der Enden des flexiblen Tubus (3) so ausgebildet ist, dass der flexible Tubus (3) direkt oder über eine zwischengeschaltete Anschlussvorrichtung an ein Beatmungssystem (9) angeschlossen werden kann.

8. Vorrichtung (1) für die retrograde Intubation nach einem der vorhergehenden Ansprüche, wobei der flexible Tubus (3) ein(e) oder mehrere nahe des distalen Endes ausgerichtete Öffnungen/Löcher umfasst, die ein Durchströmen von Luft ermöglichen.

9. Vorrichtung (1) für die retrograde Intubation nach einem der vorhergehenden Ansprüche, wobei der flexible Tubus (3) so ausgebildet ist, dass die proximalen und distalen Enden davon geschlossen und, durch Entfernen von etwas Material, geöffnet werden können.

10. Kit für die retrograde Intubation, umfassend:
- eine Vorrichtung (1) für die retrograde Intubation nach einem der vorhergehenden Ansprüche;
- einen Endotrachealtubus (7), der dazu geeignet ist in Längsrichtung in den flexiblen Tubus (3) der Vorrichtung (1) eingeführt und hineingeschoben zu werden, und/oder eine zwischengeschaltete Anschlussvorrichtung
zum direkten Anschluss des flexiblen Tubus (3) an ein Beatmungssystem (9).

11. Kit für die retrograde Intubation nach Anspruch 10, wobei der flexible Tubus (3) der Vorrichtung (1) eine geringe Längsausdehnung aufweist als die Gesamtlängserstreckung des Endotrachealtubus (7).

12. Kit für die retrograde Intubation nach Anspruch 10 oder 11, ferner umfassend ein Beatmungssystem (9), das an den flexiblen Tubus (3) und/oder den Endotrachealtubus (7) und/oder an eine zwischengeschaltete Anschlussvorrichtung angeschlossen werden kann.

13. Kit für die retrograde Intubation nach einem der Ansprüche 10 bis 12, ferner umfassend eine Manschette, die mit dem flexiblen Tubus (3) verbunden werden kann und mit einem Kanal zum Aufblasen und Entleeren der Manschette versehen ist, so dass sich der Kanal zum Aufblasen und Entleeren der Manschette in Richtung des ersten (4) oder zweiten Endes (5) des Führungsdrahtes (2) erstrecken kann.

## Revendications

1. - Dispositif (1) pour l'intubation rétrograde, comprenant un fil-guide (2) et un tube souple (3) ayant une extension longitudinale inférieure à celle du fil-guide (2), le tube souple (3) s'étendant longitudinalement parallèlement au fil-guide (2) et étant formé pour s'infléchir en suivant les inflexions du fil-guide (2) et pour se déformer radialement lors de l'introduction de celui-ci dans un trou pratiqué dans la membrane crico-thyroïdienne ou crico-trachéale d'un patient ayant une extension radiale inférieure à l'extension radiale du tube souple (3), caractérisé que le tube souple (3) est relié de manière fixe au fil-guide (2) et a une taille telle qu'un tube endotrachéal (7) peut être introduite longitudinalement et glissé dans l'intérieur de celui-ci.

2. - Dispositif (1) pour l'intubation rétrograde selon la revendication 1, dans lequel le tube souple (3) est disposé sur le fil-guide (2) de sorte que le fil-guide (2) a au moins une première partie d'extrémité (4), où le fil-guide (2) est dépourvu du tube souple (3).

3. - Dispositif (1) pour l'intubation rétrograde selon la revendication 1 ou 2, dans lequel le tube souple (3) est disposé dans une position longitudinale intermédiaire du fil-guide (2), de sorte que le fil-guide (2) a une première partie d'extrémité (4) et une seconde partie d'extrémité (5) opposée à la première (4), dans lesquelles le fil-guide (2) est dépourvu du tube souple (3) .

4. - Dispositif (1) pour l'intubation rétrograde selon l'une quelconque des revendications précédentes, dans lequel le tube souple (3) est fait d'un matériau choisi dans le groupe constitué par : le chlorure de polyvinyle (PVC), le polyuréthane, les élastomères thermoplastiques (TPE), le polyéthylène (PE), le polypropylène (PP), le polyéther block amide (PEBA), le polyamide (PA).

5. - Dispositif (1) d'intubation rétrograde selon l'une quelconque des revendications précédentes, dans lequel le tube souple (3) a une épaisseur comprise entre 50 et 500 microns.

6. - Dispositif (1) d'intubation rétrograde selon l'une quelconque des revendications précédentes, dans lequel le tube souple (3) est contraint longitudinalement au fil-guide (2) au niveau d'une paroi circonférentielle (6) de celui-ci.

7. - Dispositif (1) d'intubation rétrograde selon l'une quelconque des revendications précédentes, dans lequel au moins l'une des extrémités du tube souple (3) est formée pour permettre de raccorder le tube souple (3) à un système de ventilation (9) directement ou par un dispositif de raccordement intermédiaire.

8. - Dispositif (1) pour l'intubation rétrograde selon l'une quelconque des revendications précédentes, dans lequel ledit tube souple (3) comprend un ou plusieurs trous/ouvertures positionnés à proximité de l'extrémité distale, permettant à l'air de passer à travers.

9. - Dispositif (1) pour l'intubation rétrograde selon l'une quelconque des revendications précédentes, dans lequel le tube souple (3) est réalisé de telle sorte que les extrémités proximale et distale de celui-ci sont aptes à être fermées et ouvertes par enlèvement de matière.

10. - Kit pour l'intubation rétrograde comprenant :
- un dispositif (1) pour l'intubation rétrograde selon l'une quelconque des revendications précédentes ;
- une sonde endotrachéale (7) apte à être introduite longitudinalement et glissée à l'intérieur du tube souple (3) dudit dispositif (1), et/ou d'un dispositif de raccordement intermédiaire pour raccorder directement le tube souple (3) à un système de ventilation (9).

11. - Kit pour l'intubation rétrograde selon la revendication 10, dans lequel le tube souple (3) du dispositif (1) a une taille longitudinale inférieure à l'extension longitudinale totale du tube endotrachéal (7).

12. - Kit pour l'intubation rétrograde selon la revendication 10 ou 11, comprenant en outre un système de ventilation (9) apte à être raccordé au tube souple (3) et/ou au tube endotrachéal (7) et/ou au dispositif de raccordement intermédiaire.

13. - Kit pour l'intubation rétrograde selon l'une quelconque des revendications 10 à 12, comprenant en outre un manchon apte à être relié au tube souple (3), et muni d'un canal de gonflage-dégonflage de manchon, de telle sorte que ledit canal de gonflage-dégonflage de manchon peut s'étendre vers la première (4) ou la seconde extrémité (5) du fil-guide (2).
